# EUROPÄISCHE PATENTANMELDUNG

(11) **EP 1 364 630 A2**
(43) Veröffentlichungstag der Anmeldung: **26.11.2003**
(21) Anmeldenummer: 03010454.1
(22) Anmeldetag: 09.05.2003
(51) Int. Cl.: A61F 2/30, A61F 2/36

(54) **Positionierhilfe für Gelenkprothese**

(30) Priorität: 22.05.2002 DE 20207913 U
(71) Anmelder: ESKA Implants GmbH & Co., 23556 Lübeck (DE)
(72) Erfinder: Grundei, Hans, Dr.-Ing., 23558 Lübeck (DE)
(74) Vertreter: Fuchs Mehler Weiss & Fritzsche

(57) **Zusammenfassung**

Es wird eine Positionierhilfe bei der Herstellung einer konischen Klemmverbindung zwischen einem konischen Steckzapfen (2) und einer konischen Klemmhülse (3) in Teilen (1) einer Gelenkendoprothese beschrieben.

Sie besteht aus einer an der Basis des Steckzapfens (2) vorgesehenen Arretierungsnase (4) und wenigstens zwei radialen Ausnehmungen (5) im Öffnungsbereich der Klemmhülse (3) in dem betreffenden Gelenkteil, in welche die Arretierungsnase (4) setzbar ist.

## Beschreibung

Die Erfindung betrifft eine Positionierhilfe bei der Herstellung einer konischen Klemmverbindung zwischen einem konischen Steckzapfen und einer konischen Klemmhülse, die jeweils in Teilen einer Gelenkendoprothese vorgesehen sind.

Konische Klemmverbindungen sind in der Endoprothetik seit langem bekannt. Sie ermöglichen einen festen Sitz bei gleichzeitig gegebener Möglichkeit eines Austausches im Revisionsfall. Die Herstellung der konischen Klemmverbindung ist sehr einfach, erfordert jedoch einige Erfahrungen seitens des Operateurs, um nämlich die Winkellage des Konus im Verhältnis zur Hülse exakt einzustellen. Dies trifft insbesondere zu bei sogenannten Doppelkonen als Adapter zwischen dem einen Gelenkteil und Gelenk, insbesondere zwischen einem Hüftstiel und einer künstlichen Gelenkkugel. Ein derartiger Doppelkonus besteht aus zwei aneinander geformten Steckkonen, deren Mittelachse jeweils in einem Winkel zueinander stehen. Hiermit ist der sogenannte CCD-Winkel einstellen. Beim Einsatz beispielsweise eines Hüftstieles in den ausgefrästen Femor sucht sich dieses Gelenkteil seinen Weg in das Innere des Femors praktisch selbst. Wenn dann der Doppelkonus als Adapter in die konische Klemmverbindung gebracht werden soll, hängt es sehr davon ab, um welchen Winkel dem zuvor der Hüftstiel sich beim Eintreiben in den Femor gedreht hat.

Vor diesem Hintergrund ist es die Aufgabe der vorliegenden Erfindung, eine Positionierhilfe bereitzustellen, mit deren Hilfe ein möglichst exaktes Ausrichten des Steckkonus in der Klemmhülse ermöglicht wird.

Gelöst wird diese Aufgabe durch eine Positionierhilfe mit den Merkmalen des Anspruchs 1. Vorteilhaft Weiterbildungen ergeben sich aus den Unteransprüchen.

Sinngemäß besteht die Positionierhilfe aus einer an der Basis des Steckzapfens vorgesehenen Arretierungsnase und aus wenigstens zwei radialen Ausnehmungen im Öffnungsbereich der Klemmhülse in dem betreffenden Gelenkteil, in welche die Arretierungsnase setzbar ist. Die Dimensionierung ist nun so, dass die Arretierungsnase in eine der Ausnehmungen eingesetzt wird, wenn die konische Klemmverbindung schon weitgehend hergestellt ist. Lediglich ein letztes Eintreiben des Zapfens in die Hülse erzeugt den starken konischen Klemmsitz. Dann liegt die Arretierungsnase vollständig in der betreffenden Ausnehmung.

Gemäß einer besonderen Ausführungsform ist die Arretierungsnase im Bereich des Überganges von einem konischen Klemmzapfen zu einem angeformten zweiten konischen Klemmzapfen eines als Doppelkonus ausgebildeten Adapters vorgesehen. Derartige Doppelkonen sind für sich genommen bekannt. Die Hauptachsen der beiden aneinander angeformten Konen stehen in einem vorgegebenen Winkel zueinander. Kommt dieser Doppelkonus im Rahmen der Implantation eines künstlichen Hüftgelenkes zum Einsatz, kann mit der Drehung des Doppelkonus um die Achse des einen Steckzapfens der sogenannte CCD-Winkel eingestellt werden. Die erfindungsgemäße Positionierhilfe gestattet dann eine Kompensation einer etwaigen Eigenrotation des Hüftstiels beim Eintreiben des Stieles in den Femorknochen.

Die Ausnehmungen in den Öffnungsbereich der Klemmhülse sind in einem Winkelbereich von 90° um die Mittenachse der konischen Klemmhülse angeordnet. Der linke Bereich erlaubt ausreichende Einstellungsmöglichkeiten, ohne dass die Stabilität des Gelenkteils an seinem Rand unzulässigerweise leiden würde.

Die Erfindung wird anhand eines Ausführungsbeispiels gemäß Zeichnungsfiguren beispielhaft näher erläutert. Hierbei zeigt:
- Fig. 1: eine schematische Schnittansicht durch ein Teil einer künstlichen Hüftgelenksendoprothese,
- Fig. 2: eine schematische Ansicht eines Doppelkonus als Adapter in dem Implantat nach Fig. 1 und
- Fig. 3: eine Aufsicht auf das Teil der Gelenkendoprothese gemäß Fig. 1 ohne Doppelkonus.

Nachfolgend bezeichnen gleiche Bezugszeichen gleiche Teile.

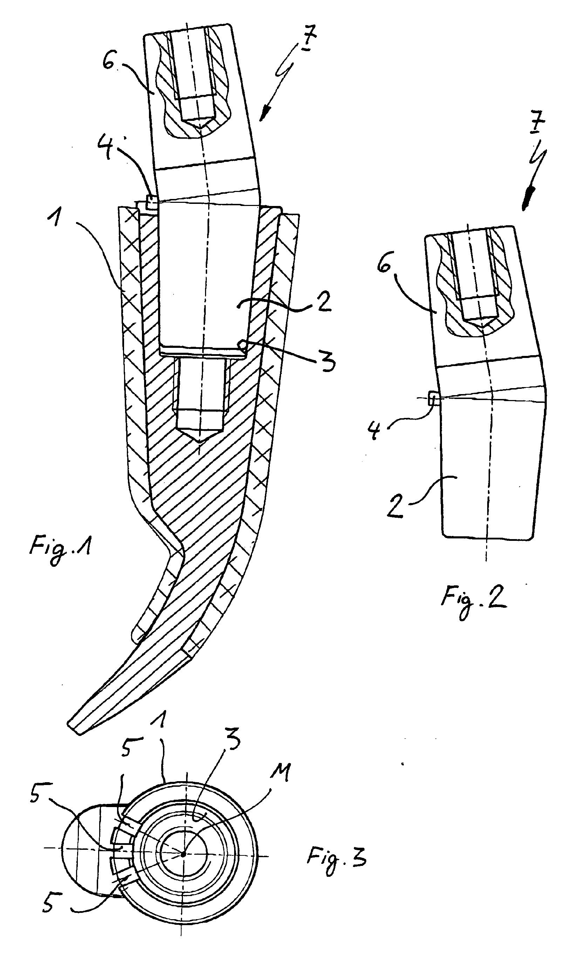

Einen ersten Überblick verschafft Fig. 1. Diese zeigt eine schematische Schnittansicht durch ein Teil 1 einer Gelenkendoprothese vorliegend einer Hüftgelenksendoprothese.
Das Teil 1 als solches ist bekannt aus der DE-C-19720493. In das Teil 1 ist eine konische Klemmhülse eingearbeitet, in welcher ein konischer Steckzapfen 2 eines Adapters 7 in Form eines Doppelkonus sitzt. Der Doppelkonus 7 als Adapter ist so ausgebildet, dass an dem einen Steckzapfen 2 ein weiterer Steckzapfen 6 angeformt ist, und zwar an deren beider Basis.

An der Basis der Steckzapfen 2 und 6 ist eine Arretierungsnase 4 angeformt. Diese Arretierungsnase 4 ist Teil der Positionierhilfe. Diese besteht darüber hinaus aus radialen Ausnehmungen 5 (Fig. 3) im Öffnungsbereich der Klemmhülse 3 in dem Teil 1 des Hüftstiels. Beim Hineinstecken des konischen Steckzapfens 2 in die Klemmhülse 3 kann der Operateur den Adapter 7 so orientieren, dass die Arretierungsnase 4 in eine der drei Ausnehmungen 5 eingreift. Die Dimensionen sind so gewählt, dass die Arretierungsnase 4 voll in eine Ausnehmung 5 greift, wenn der konische Klemmsitz zwischen dem Steckzapfen 2 und der Klemmhülse 3 hergestellt ist.

## Patentansprüche

1. Positionierhilfe bei der Herstellung einer konischen Klemmverbindung zwischen einem konischen Steckzapfen (2) und einer konischen Klemmhülse (3) in Teilen (1) einer Gelenkendoprothese, bestehend aus einer an der Basis des Steckzapfens (2) vorgesehenen Arretierungsnase (4) und wenigstens zwei radialen Ausnehmungen (5) im Öffnungsbereich der Klemmhülse (3) in dem betreffenden Gelenkteil, in welche die Arretierungsnase (4) setzbar ist.

2. Positionierhilfe nach Anspruch 1, bei der die Arretierungsnase (4) im Bereich des Überganges von einem konischen Klemmzapfen (2) zu einem angeformten zweiten konischen Klemmzapfen (6) eines als Doppelkonus ausgebildeten Adapters (7) vorgesehen ist.

3. Positionierhilfe nach Anspruch 1 oder 2, bei der die Ausnehmungen (5) in einem Winkelbereich von 90 ° um die Mittenachse (M) der konischen Klemmhülse (3) angeordnet sind.
